# EUROPÄISCHE PATENTSCHRIFT

(11) **EP 1 307 174 B1**
(45) Veröffentlichungstag und Bekanntmachung des Hinweises auf die Patenterteilung: **05.01.2005**
(21) Anmeldenummer: 01976059.4
(22) Anmeldetag: 06.08.2001
(51) Int. Cl.: A61K 6/093

(54) **ADHESIVE SILICONFÜLLUNGS- UND BEFESTIGUNGSMATERIALIEN**
ADHESIVE SILICON FILLING AND FIXING MATERIALS
PRODUITS D'OBTURATION AU SILICIUM ET DE FIXATION ADHESIFS

(30) Priorität: 07.08.2000 DE 10039297; 17.08.2000 DE 10040725
(43) Veröffentlichungstag der Anmeldung: 07.05.2003
(73) Patentinhaber: S & C Polymer Silicon- und Composite-Spezialitäten GmbH, 25335 Elmshorn (DE)
(72) Erfinder: ENGELBRECHT, Jürgen, 22607 Hamburg (DE)
(74) Vertreter: Forstmeyer, Dietmar, Dr. rer. nat., Dipl.-Chem.
(86) Internationale Anmeldenummer: PCT/EP2001/009071
(87) Internationale Veröffentlichungsnummer: WO 2002/011680

(56) Entgegenhaltungen:
- EP-A- 0 632 060
- EP-A- 0 864 312
- WO-A-99/03424
- US-A- 3 127 363

## Beschreibung

Die Erfindung betrifft die Bestecke (Kit of parts), die (Co-) Polymere mit Hydrogensiloxangruppen, einen Katalysator, eine Vinylverbindung und eine Hydrosilylverbindung enthalten. Die Bestecke können als dauerhafte oder provisorische weich- oder hartelastische Befestigungs- oder Füllungsmaterialien an Zahn- oder Knochensubstrat eingesetzt werden.

Im dentalen Bereich werden häufig Silicone als Abformmassen verwendet. Insbesondere können kondensationsvernetzende hydroxylgruppenhaltige Polysiloxane (vernetzt z.B. durch Orthosilicate Si(OR)₄ oder Silane RSi(OR)₃) oder additionsvernetzende vinylgruppenhaltige Polysiloxane (vernetzt durch hydrosilylgruppenhaltige Polysiloxane) eingesetzt werden. Im Bereich der dentalen Füllungsmaterialien oder Zemente sind sie jedoch wenig bis kaum bekannt oder genutzt.

Die DE 3 915 592 beschreibt einen Dentalzement bestehend aus einem säuregruppenhaltigen Siliconöl, welches mit Carboxylgruppen modifiziert ist. Das Vermischen mit Metalloxiden oder Metallhydroxiden führt zu einem hartem zementähnlichen Verschlußmaterial, speziell zum Zahnwurzelkanalverschluß.

Die US 3 082 520 A offenbart eine Mischung zum Füllen von Zahnwurzelkanälen basierend auf hydroxylgruppenhaltigem Polysiloxan, welches mit Organotriacyloxysilan vernetzt wird. Der Typ des kondensationsvenetzenden Silicons bedingt eine Abspaltung von Essigsäure und führt zu einer Schrumpfung, die nachteilig für Füllungsmaterialien ist.
Einen ähnlichen Weg beschreibt die US 3 127 363 A, die ebenfalls ein kondensationsvernetzendes Siliconsystem beschreibt.

Die DE 197 09 531 & EP 0864 312 beschreibt ein additionsvernetzendes Polysiloxansystem zum Füllen von Wurzelkanälen, welches gegenüber den vorhergehenden Anmeldungen den Vorteil geringerer Schrumpfung aufzeigt.

Alle vorhergehenden Formulierungen haben jedoch einen sehr großen Nachteil: sie zeigen keinerlei Haftung an Zahn- oder Knochensubstrat.

In der PCT/EP 0001042 und EP 0 632 060 A1 werden Haftvermittler aus Copolymeren enthaltend Hydrogensiloxangruppen sowie Lösungen daraus beschrieben, die die Haftung von additionsvernetzenden Siliconen z.B. auf Prothesenmaterial bewirken können.

In beiden Schriften bewirken Copolymere, die z.B. Bruchstücke polymerisierter Methacrylate und Bruchstücke hydrosilylhaltiger Polysiloxane enthalten, die haftende Wirkung sowohl zum anlösbaren Kunststoff, als auch zum additionsvernetzenden Silicon.

Haftvermittler der PCT/EP 0001042 haften unerwartet auch an Zahnhartsubstanz.

Aufgabe der vorliegenden Erfindung war es, Mischungen zu finden, die in der Lage sind, an Zahn - oder Knochensubstrat zu haften, und als weich- oder hartelastische Klebezemente oder Füllungsmaterialien verwendet werden können.

Erfindungsgemäß wurde die Aufgabe gelöst durch Verwendung von einem Besteck, das
a) ein Copolymeres mit Hydrogensiloxangruppen,
b) einen Katalysator,
c) eine Vinylverbindung, und
d) eine Hydrosilylverbindung
enthält,
dadurch gekennzeichnet, daß das Copolymere mit Hydrogensiloxangruppen a) ein Copolymeres von SiH-Gruppen haltigen Siloxanen mit ungesättigten Verbindungen ist, oder
daß das Copolymere mit Hydrogensiloxangruppen a) ein Copolymeres von ungesättigten Verbindungen ist, dem SiH-Gruppen haltige Siloxane aufgepfropft sind.

Ein derartiges Besteck kann zur Herstellung von dauerhaften oder provisorischen weich- oder hartelastischen Klebezementen oder Füllungsmaterialien an Zahn oder Knochensubstrat eingesetzt werden.

Bevorzugt weist das Besteck zwei Unterbestecke auf, wobei
Unterbesteck 1
   a) das Copolymere mit Hydrogensiloxangruppen, und
Unterbesteck 2
   b) den Katalysator,
   c) die Vinylverbindung, und
   d) die Hydrosilylverbindung
enthält.

Das Unterbesteck 1 (auch "der Primer" genannt) enthält als wichtige auf Zahn- und auf Knochensubstrat haftende Substanz Copolymere, d.h. Polymere oder Copolymere, im weiteren auch nur Polymere genannt, mit Hydrogensiloxangruppen. Das Grundgerüst dieser Polymere können z.B. Copolymere aus Methylmethacrylat und Allylmethacrylat sein, denen Hydrogensiloxan aufgepropft ist. Es können auch Copolymere sein wie sie ausführlich in der EP 0 632 060 A1 beschrieben sind, oder auch, besonders bevorzugt, Copolymere, die durch direkte Polymerisation von Hydrogensiloxanen gewonnen sind, wie sie in der PCT/EP 0001042 und in der japanischen Offenlegungsschrift HEI 10-25322 beschrieben sind.

Die SiH-Gruppen der Hydrogensiloxaneinheiten vermögen offenbar mit den POH-Gruppen des Apatiths der Zahnsubstanz zu reagieren, so daß ohne vorherige mechanische Aufrauhung (Ätzung) eine Bindung des Lackes zur Zahnsubstanz auf chemischen Wege zustande kommt.

Zusätzlich können erfindungsgemäß verwendete Primer bis zu 10, vorzugsweise bis zu 1 Gewichtsteil einer Vinylverbindung, bis zu 500 ppm, vorzugsweise bis zu 50 ppm Platinkatalysator und bis zu 80, vorzugsweise bis zu 20 Gewichtsteile Polymer(e) ohne Hydrogensiloxangruppen enthalten.

Zusätzlich können aber auch bis zu 3, vorzugsweise bis zu 0,3 Gewichtsteile von in additionsvernetzenden Siliconen verwendeten Hydrogenpolysiloxane zugegeben sein.

Es kann nützlich sein gegebenenfalls einen Katalysator zuzugeben, der die Reaktionen SiH + POH oder SiH +Vinylsiloxan zu katalysieren vermag. Mögliche Katalysatoren sind Verbindungen des Platins oder Palladiums sowie Legierungen von diesen.

Besonders geeignet sind Platin(0)-Verbindungen, die vom Platinhexachloroplatinat ausgehend hergestellt sind und welche üblich sind in Systemen additionsvernetzender Vinylpolysiloxane / Hydrogenpolysiloxane. Beispiele für Katalysatoren sind Ptₓ(Divinyltetramethyldisiloxan)_{y} des Platin(0).

Gegebenenfalls zugesetzte Vinylverbindungen sollten bevorzugt Divinylverbindungen sein. Aber auch Verbindungen mit mehr als zwei Vinylgruppen können sinnvoll sein, so z.B., wenn ein hoher Vernetzungsgrad erreicht werden soll. Bevorzugte Divinyl-Verbindungen sind Divinylether wie Divinylether von Triethylenglycol oder Cyclohexandiol oder Divinylpolysiloxane wie sie in additionsvernetzenden Siliconen verwendet werden oder auch Divinyltetramethyldisiloxan. Auch die Zugabe von Methacrylatverbindungen kann vorteilhaft sein.

Verbindungen mit Vinylgruppen können gegebenenfalls auch in vorteilhafter Weise Säuregruppen tragen, die zusätzlich zu einer Haftung auf Zahn- oder Knochensubstanz beitragen können. Beispielhafte Säuregruppen sind -COOH, -Phosphat, Phosphonat.

Gegebenenfalls zugesetzte andere Hydrogensiloxane sind vorzugsweise Polysiloxane mit mindestens zwei, vorzugsweise drei SiH-Gruppen.

Erfindungsgemäß verwendete Primer können zusätzlich auch Polymere enthalten, die keine SiH-Gruppen aufzeigen. Beispiele dafür sind Polymethacrylate, Polycarbonate, Polyester und ähnliche Polymere sofern sie in derMischung löslich und mit dem System verträglich sind.

Ebenfalls können erfindungsgemäße Mischungen leicht flüchtige Lösungsmittel enthalten, die es ermöglichen den Lack als sehr dünnen Film aufzutragen. Als Lösungsmittel eignen sich besonders leichtflüchtige inerte Lösungsmittel wie halogenierte oder nichthalogenierte aliphatische oder aromatische Kohlenwasserstoffe, Ether, Ketone, Ester oder cyclische Siloxane.

Auch können Verbindungen oder Mittel zugesetzt werden, die biozide oder pharmazeutische Wirkungen wie z.B. antibacterielle oder entzündungshemmende Wirkungen aufzeigen. Biozide Verbindungen oder Mittel töten Bakterien, Viren und/oder Pilze, während biostatische Verbindungen oder Mittel das Wachstum von Bakterien, Viren und/oder Pilzen hemmen.

Die härtenden Mischungen (Unterbesteck 2), die in einem zweiten Schritt nach Auftragen des Haftprimers verwendet werden, sind additionsvernetzende Silicone, deren Basiskomponenten Vinyl-, und Hydrosilylverbindungen und Katalysatoren sind. Sie können Füllstoffe, Thixotropierungsmittel, Verstärker, Pigmente, Farbstoffe, Weichmacher, Stabilisatoren, Emulgatoren, Hydrophilierungsmittel, biozide oder pharmazeutische Zusätze haben. Die Zusätze richten sich nach den zu erreichenden Eigenschaften des Befestigungs- oder Füllungsmaterials. So können durch Füllstoffe wie feinteilige Mehle von Quarz, Calciumcarbonat, Talkum etc., wie sie auch in addtionsvernetzenden Abformsiliconen verwandt werden, eine hohe Härte erreicht werden. Thixotropierungsmittel wie pyrogene Kieselsäuren oder Ethylcellulose, die das Fließverhalten oder die Reißfestigeit steuern, Pimente oder Farbstoffe, die ein Zahn- oder knochenähnlichen Aussehen bewirken, Weichmacher, Stabilisatoren, Emulgatoren und Hydrophilierungsmittel, die für die richtigen Handhabungs- und Stabilitätseigenschaften sorgen und biozide oder pharmazeutische Zusätze, die der besonderen biologischen Umgebung von Zahn- und Knochensubstanz Rechnenung tragen.

Die im Unterbesteck 2 eingesetzten Katalysatoren sind vorzugsweise Platinkatalysatoren und die Vinylverbindungen sind vorzugsweise Vinylsiloxanverbindungen; die Hydrosilylverbindungen sind vorzugsweise Hydrogensiloxanverbindungen.

Die erfindungsgemäßen Bestecke aus den oben genannten Primern und härtenden Mischungen eignen sich hervorragend als auf Zahnoder Knochensubstanz haftende weich- oder hartelastische Verschlußmaterialen. Die Art der Weich- oder Hartelastizität kann über die härtende Mischung eingestellt werden, ebenso die viscoelastischen Eigenschaften oder Aushärtezeiten. So kann es vorteilhaft sein, die härtenden Mischungen knetmassenartig einzustellen, sodaß z.B. im Falle der provisorischen Füllung einer Zahnkavität nach Applikation des Primers eine zahnfarbene Mischung von additionsvernetzenden Siliconknetmassen nach Zusammenkneten in die Kavität gedrückt und unter Aufbeißen zu einer hartelastischen Füllung ausgehärtet lassen wird. Es kann aber auch vorteilhaft sein, eine zahnfarbene dünnfließende Mischung aus einer selbstmischenden Applikations-Doppelkartusche direkt in eine mit dem Primer vorbehandelte Kavität fließen zu lassen, sie mit einer vorhergefertigten Occlusionsmatrix z.B. aus thermoplastischer Folie zu verschließen und sie aushärten zu lassen. Die Härte kann die gleiche sein wie bei der knetbaren Anwendungsform. Durch die Haftwirkung des Primers verbleiben die Füllungen in der Kavität. Ohne Anwendung des Haftprimers bilden sich schnell Randspalte durch die elastischen Bewegungen bei Kauen. Abgesehen von den Problemen der Kontamination in den Randspalten fällt die Füllung ohne Haftprimer schnell wieder heraus.

Die Haftkraft des Primers kann voreingestellt sein. Mehr oder weniger an SiH-Haftgruppen am Copolymer ergibt mehr oder weniger Haftkraft des Primers. Die Haftkraft kann aber auch beim Anwenden durch nur punktuelle Applikation oder bestreichen nicht aller Flächen reduziert werden.

Provisorische Füllungen, appliziert mit einem Primer verminderter Haftkraft, und verwendet mit härtenden Mischungen erfindungsgemäßer Art sind preiswert, schnell appliziert und können im Notfall auch außerhalb der üblichen zahnärztlichen Behandlungsstuhlumgebung gelegt werden. Sie haften ausreichend gut um nicht von selbst herauszufallen und sind bei Bedarf mit einem Handinstrument leicht wieder entfernbar.

Bei Wurzelkanalfüllungen auf Basis additionsvernetzender Silicone kann vorteilhaft die zusätzliche Verwendung des Haftprimers die an sich schon gute Versiegelungseigenschaft dauerhaft unterstützen.

In Fällen von notwendigen Auffüllungen fehlender Knochensubstanz ohne Anforderungen an hoher Härte und Stützwirkung kann es sinnvoll sein, dieses dauerelastisch zu bewerkstelligen.

Insbesondere wenn z. B. ein Verschluß eines Knochenhohlraumes oder der Öffnung eines solches gefordert ist, kann die erfindungsgemäße Applikation von Haftprimer und härtender Siliconmischung zu bisher nicht erreichbaren Lösungen führen. In einer anderen Anwendung können druckgefährdete Knochenstellen mit Silicon abgepuffert werden, auch provisorisch.

Auch als elastische Befestigungsmaterialien können erfindungsgemäße Verwendungen neue Wege beschreiten lassen.

So können Inlays oder Onlays aus Metall, Keramik oder glasgefüllten Methacrylatcomposits nach Silanisierung der Oberflächen der Inlays/Onlays mit aktivierten Silanen wie z.B. RSi(OH)₃ (wobei R = Methyl, Ethyl, Vinyl, Methacryl, Allyl) und Applikation des Haftprimers im Kavitätenbereich auf Dentin und Schmelz dauerhartelastisch mithilfe von härtenden Vinylsiliconen belastbar zementiert werden. Die Elastizität des Zementes läßt Spannungen durch occlusale Spitzenbelastung der Onlay-/Inlayoberflächen abpuffern. Besonders die Randspaltfreiheit durch Verwendung der extrem schrumpfarmen additionsvernetzenden Silicone eröffnet neue Möglichkeiten.

Auch die Verwendung als Bracketadhesiv zur orthodontischen Regulierung von Zahnstellungen ergibt neue Möglichkeiten: Durch die Adhesivität des Primers auf Zahnschmelz und auf Unedelmetallbrackets kann eine zwischen Zahn und Bracket befindliche elastische Siliconzementschicht eine schonendere Übertragung der Spannkraft der gespannten Zugbänder des Bracketsystems auf die zu versetzenden Zähne ergeben.

Ähnliches gilt für erfindungsgemäß verwendete Primer und härtende Mischungen als Zement für Splinte als Halteapparat für paradontal geschädigte und gelockerte Zähne. Die erfindungsgemäße Verwendung ermöglicht eine Haltehilfe der Nachbarzähne, belastet diese aber nicht im gleiche Maße wie den gestützten Zahn.

Besonders neue Möglichkeiten eröffnen die erfindungsgemäßen Verwendungen von Primer und härtenden Mischungen als elastische Knochenklebezemente. So ermöglichen Applikationen eines erfindungsgemäßen Primers auf je einem zu klebenden Knochenteil und einer Schicht von Bruchteilen von Millimetern bis einigen Millimetern, gegebenenfalls bis Zentimetern eine elastische Verbindung zwischen zwei Knochenteilen. Die erfindungsgemäße Verwendung von Primer und härtenden Mischungen
kann somit gegebenenfalls die Funktion von Knorpel übernehmen. Die gute Bioverträglichkeit von additionsvernetzenden Siliconen ist bekannt und damit prinzipiell für einen solchen Zweck geeignet.

Auch elastische Verklebungen von Keramik und Knochen sind auf dies Weise möglich. Die zur erfindungsgemäßen Verwendung kommenden Primer können auch gut auf Keramik haften, die auf der Oberfläche ausreichend SiOH-Gruppen aufweisen und hervorragend mit den SiH- Gruppen des Primers reagieren können. So können biokeramische Gelenke gegebenenfalls durch eine Zwischenschicht Silicon abgepuffert werden und somit nicht so leicht zum Bruch neigen, auch bei hohen Schockbelastungen.

### Beispiele

### Beispiel 1

### Provisorische Zahnfüllung

Fällt ein Goldinlay aus einer Zahnkavität während kein Zahnarzt erreichbar ist, so kann das erfindungsgemäße Besteck auch von Laien verwendet werden: Eine mitgeführte Menge additionsvernetzender Siliconabformmasse Formasil A Putty (Kulzer, Australien, Materialfarbe ca. beige) und ein ebenfalls mitgeführter farbloser Primer nach Beispiel 2 der PCT/EP 00/01042, der als Haftsubstanz ein Polymerisat aus Hydrogensiloxan und Ethylmethacrylat enthält, können als provisorische Füllung dienen. Die leere Kavität wird nach Spülen mit Wasser und Trocknen mit kleinen Stückchen eines Papiertaschentuches mithilfe ebenfalls kleinen Papiertaschentücherstückchen und eines Zahnstochers mit dem Primer unter Tupfen benetzt und trocknen gelassen (das Lösungsmittel des Primers verdampft schnell). Eine kleine Menge angemischten Formasil A Puttys wird vorsichtig in die Kavität gedrückt, ein Stückchen Plastikfolie (aus der Packungsfolie der Papiertaschentücher) zwischen Gegenkiefer und Siliconfüllung gelegt und sodann der Kiefer unter leichtem seitlichen Hin- und Herbewegen geschlossen. Nach 3 Minuten ist die Füllung ausgehärtet. Überhänge können leicht mithilfe eines Fingers weggedrückt oder mit einem Zahnstocher entfernt werden. Die provisorische Füllung verbleibt haftend in der Kavität. Sie ist hermetisch verschlossen, es kann sofort wieder gegessen werden. Nach einigen Tagen kann die provisorische Füllung von einem Zahnarzt leicht mit einem Handinstrument gebröselt und mit Unterstützung eines rotierenden Instrumentes leicht restlos entfernt werden. Die Kavität kann jetzt fachmännisch versorgt werden.

### Beispiel 2

### Nicht-haftende Zahnwurzelkanalzementierung mit Silicon (nicht erfindungsgemäß)

Für einen provisorischen Verschluß werden Guttaperchaspitzen zusammen mit dem Wurzelfüllungsmaterial RoekoSeal (Roeko, Deutschland), formuliert auf Basis additionsvernetzender Vinylsilicone, in einen geöffneten Wurzelkanal eingebracht und die Kavität mit einem provisorischen Cement Cavit (Espe, Deutschland) provisorisch verschlossen. Nach 10 Tagen wird das Cavit entfernt. Die Guttaperchaspitze kann mitsammt dem RoekoSeal in einem Stück leicht herausgenommen werden. Der Wurzelkanal kann inspiziert und erneut provisorisch oder permanent verschlossen werden.

### Beispiel 3

### Haftende Zahnwurzelkanalzementierung mit Silicon

Für einen permanenten Verschluß wird der Wurzelkanal vor dem Verschluß mit Guttapercha/RoekoSeal mit der Haftprimerlösung wie im Beispiel 1 verwendet gespült, 3 Minuten einwirken gelassen, die überschüssige Lösung mit Papierspitzen ausgetupft und mit einem kurzen Luftstrom getrocknet. Danach werden Guttaperchaspitzen zusammen mit dem additionsvernetzenden Wurzelfüllungsmaterial RoekoSeal (Roeko, Deutschland) eingebracht. Die Kavität wird wieder mit Cavit (Espe,Deutschland) provisorisch verschlossen. Das RoekoSeal härtet aus. Am Folgetag das Cavit entfernt. Die Cavität kann mit Komposit gefüllt werden. Auch wenn der obere Teil der RoekoSeal-Füllung mitgefräßt wird, so verbleibt der Rest der Siliconfüllung haftend im Wurzelkanal, er kann nicht versehentlich mit herausgezogen werden.

### Beispiel 4

### Provisorische Zementierung einer provisorischen Krone

Eine nur grob passende provisorische Fertigkrone aus Polycarbonat wird in ihrer Innenfläche mit der Haftprimerlösung aus Beispiel 1, die entsprechend PCT/EP 00/01042 sehr gut auf diesem Kunstoff haftet, dünn eingepinselt und getrocknet. Der präparierte Zahnstumpf, auf den die provisorische Krone vorübergehend aufgebracht werden soll, wird nur an wenigen Punkten mit der Haftprimerlösung touchiert. Ein dünnfließendes additionsvernetzendes Hartsilikon, welches normalerweise als Modellsilicon für die Inlay-Technik in direkter Methode dient und welches Zahnfarben eingefärbt ist, DieFlex (Danville Materials, USA), wird in die vorbehandelte Fertigkrone gegeben und auf den Stumpf aufgesetzt. Nach 2 Minuten ist das Hartsilicon, nun als Zement verwendet, ausgehärtet. Die Überschüsse lassen sich leicht mit einem Handinstrument wegbrechen.

### Beispiel 5

### Dauerhafte Zementierung einer (permanenten) Einzelkrone

Eine sehr gut passende Metallkeramik-Einzelkrone, aufgebaut auf in ihrem Metallteil aus Chromkobaltlegierung, wird ebenfalls in ihrer Innenfläche mit der Haftprimerlösung aus Beispiel 1, die entsprechend PCT/EP 00/01042 auch sehr gut auf Unedelmetallen haftet haftet, dünn eingepinselt und getrocknet. Der präparierte Zahnstumpf wird nun über die gesamte zu überkappende Stumpffläche mit der Haftprimerlösung behandelt und gut getrocknet. Ein mittelfließendes, aber unter Druck zu extrem dünnen Schichten ausfließendes, tranparentes additionsvernetzendes Silikon, welches normalerweise als transparentes Bißregistrat oder als transparente Matrix zum Lichthärten von Compositrestaurationen dient, Clearbite (Discus Dental, USA), wird in die vorbehandelte Krone gegeben und auf den Stumpf aufgesetzt. Nach 3 Minuten ist das Hartsilicon, nun als Zement verwendet, ausgehärtet. Die Überschüsse werden am Kronenrand vorsichtig weggeschnitten. Die Krone sitzt sehr fest. Der Zement hält die Krone eine Spur elastisch, was bei Keramikkronen im Falle großer punktförmiger Belastungen gegebenenfalls ein Absplittern von Keramik von Metallgerüst verhindern kann.

### Beispiel 6

### Elastische Knochenzementierung

Zwei frische Rippenknochen eines Schweines werden je an einem Ende senkrecht geschnitten, an den Rändern ca. 3 mm der wagerechten Kante mit einem rotierenden Fräser schräg angeschliffen und abtupfend getrocknet. Die geschnittenen und beschliffenen Flächen werden mithilfe eines Dentalpinsels zweimal dünn mit der Haftprimerlösung aus Beispiel 1 bestrichen und getrocknet. Um beide Teile der zu verbindenden Rippen, die in einem Abstand von 6 mm gehalten werden, wird eine ca. 3 cm breite transparente Folie geklebt, die auf der Oberseite mit zwei ca. 2mm großen Öffnungen versehen wird. Der so gewonnene Raum zwischen beiden Rippenteilen wird mit dem vorhererwähnten transparenten additionsvernetzenden Silicon Clearbite gefüllt, indem dieses mit einen statischen Mischer und einem sogenannten Intraoral-Tip, wie sie für Applikationen zur Abdrucknahme im zahnärztlichen Bereich üblich sind, durch die erste Lochöffnung bis zum Boden des zu füllenden Hohlraumesgeführt und von dort die Masse auffüllen läßt, bis sie aus der zweiten Öffnung herauskommt. Auf diese Weise ist der Raum zwischen den Knochenteilen aufgefüllt. Das Silicon ist binnen 3 Minuten ausgehärtet. Nach Abnahme der hilfsweise verwendeten Klebefolie sind beide Teile elastisch verbunden. Überschüsse werden mit einem Skalpell weggenommen. Eine Lagerung in Wasser bei 40°C läßt auch nach 4 Wochen keine Schwächung des Verbundes oder der Elastizität erkennen.

## Patentansprüche

1. Besteck, das
a) ein Copolymeres mit Hydrogensiloxangruppe
b) einen Katalysator,
c) eine Vinylverbindung, und
d) eine Hydrosilylverbindung
enthält, **dadurch gekennzeichnet, daß**
das Copolymere mit Hydrogensiloxangruppen a) ein Copolymeres von SiH-Gruppen haltigen Siloxanen mit ungesättigten Verbindungen ist, oder
daß das Copolymere mit Hydrogensiloxangruppen a) ein Copolymeres von ungesättigten Verbindungen ist, dem SiH-Gruppen haltige Siloxane aufgepfropft sind.

2. Besteck nach Anspruch 1, das zwei Unterbestecke aufweist, wobei
Unterbesteck 1
a) das Copolymere mit Hydrogensiloxangruppen, und
Unterbesteck 2
b) den Katalysator,
c) die Vinylverbindung, und
d) die Hydrosilylverbindung
enthält.

3. Besteck nach einem der Ansprüche 1-2, **dadurch gekennzeichnet, daß** der Katalysator b) ein Platinkatalysator ist.

4. Besteck nach einem der Ansprüche 1-3, **dadurch gekennzeichnet, daß** Unterbesteck 1
e) eine von a) verschiedene Hydrosilylverbindung enthält.

5. Besteck nach Anspruch 4, **dadurch gekennzeichnet, daß** die Hydrosilylverbindung e) ein Polysiloxan mit mindestens zwei, vorzugsweise drei SiH-Gruppen ist.

6. Besteck nach einem der Ansprüche 1-5, **dadurch gekennzeichnet, daß** die Vinylverbindung c) eine Vinylsiloxanverbindung und die Hydrosilylverbindung d) eine Hydrogensiloxanverbindung ist.

7. Besteck nach einem der Ansprüche 1-6, **dadurch gekennzeichnet, daß** Unterbesteck 1
f) eine Vinylverbindung, und
g) einen Katalysator
enthält.

8. Besteck nach einem der Ansprüche 1-7, **dadurch gekennzeichnet, daß** die Vinylverbindung f) ein Vinylsiloxan, ein Vinylether oder eine Verbindung mit Allylgruppen ist.

9. Besteck nach Anspruch 7 oder 8, **dadurch gekennzeichnet, daß** der Katalysator g) ein Platin- oder Palladiumkatalysator oder ein Katalysator aus einer Legierung der beiden Metalle ist.

10. Besteck nach einem der Ansprüche 1-9, **dadurch gekennzeichnet, daß** Unterbesteck 1
h) ein (Co-)Polymeres ohne Hydrosilylgruppen enthält.

11. Besteck nach einem der Ansprüche 1-10, **dadurch gekennzeichnet, daß** Unterbesteck 1
i) ein Lösungsmittel enthält.

12. Besteck nach einem der Ansprüche 1-11, **dadurch gekennzeichnet, daß** das Lösungsmittel i) ein leicht flüchtiges Lösungsmittel ist.

13. Besteck nach einem der Ansprüche 1-12, **dadurch gekennzeichnet, daß** Unterbesteck 1 und/oder 2 biostatische, biozide oder andere pharmazeutische Verbindungen oder Mittel enthalten.

14. Besteck nach einem der Ansprüche 1-13, **dadurch gekennzeichnet, daß** Unterbesteck 2 Füllstoffe, Thixotropierungsmittel, Verstärker, Pigmente, Farbstoffe, Weichmacher, Stabilisatoren, Emulgatoren und/oder Hydrophobierungsmittel enthält.

15. Verwendung eines Bestecks nach einem der vorstehenden Ansprüche als haftendes elastisches Verschlußmaterial.

16. Verwendung eines Bestecks nach einem der Ansprüche 1-14 als haftender Wurzelkanalzement.

17. Verwendung eines Bestecks nach einem der Ansprüche 1-14 als haftendes elastisches Zahnfüllungsmaterial.

18. Verwendung eines Bestecks nach einem der Ansprüche 1-14 als haftendes elastisches provisorisches Zahnfüllungsmaterial.

19. Verwendung 'eines Bestecks nach einem der Ansprüche 1-14 als elastisches Knochenfüllmaterial.

20. Verwendung eines Bestecks nach einem der Ansprüche 1-14 als elastische Befestigung.

21. Verwendung eines Bestecks nach einem der Ansprüche 1-14 als haftender elastischer Zement.

22. Verwendung eines Bestecks nach einem der Ansprüche 1-14 als haftender elastischer provisorischer Zement.

23. Verwendung eines Bestecks nach einem der Ansprüche 1-14 als elastisches Bracketadhesiv.

24. Verwendung eines Bestecks nach einem der Ansprüche 1-14 als elastische Splintbefestigung.

25. Verwendung eines Bestecks nach einem der Ansprüche 1-14 als elastischer Knochenklebezement.

## Claims

1. A kit of parts comprising
a) a copolymer having hydrogen-siloxane groups,
b) a catalyst,
c) a vinyl compound, and
d) a hydrosilyl compound,
**characterized in that** the copolymer having hydrogen-siloxane groups a) is a copolymer of siloxanes containing SiH groups with unsaturated compounds, or the copolymer having hydrogen-siloxane groups a) is a copolymer of unsaturated compounds onto which are grafted siloxanes having SiH groups.

2. The kit of parts according to Claim 1, which comprises two sub-kits of parts, whereby
the sub-kit of parts 1 contains
a) the copolymer having hydrogen-siloxane groups, and
the sub-kit of parts 2 contains
b) the catalyst,
c) the vinyl compound, and
d) the hydrosilyl compound.

3. The kit of parts according to one of Claims 1-2, **characterized in that** the catalyst b) is a platinum catalyst.

4. The kit of parts according to one of Claims 1-3, **characterized in that** the sub-kit of parts 1 contains
e) a hydrosilyl compound different from a).

5. The kit of parts according to Claim 4, **characterized in that** the hydrosilyl compound e) is a polysiloxane having at least two, preferably three SiH groups.

6. The kit of parts according to one of Claims 1-5, **characterized in that** the vinyl compound c) is a vinylsiloxane compound and the hydrosilyl compound d) is a hydrogen-siloxane compound.

7. The kit of parts according to one of Claims 1-6, **characterized in that** the sub-kit of parts 1 contains
f) a vinyl compound, and
g) a catalyst.

8. The kit of parts according to one of Claims 1-7, **characterized in that** the vinyl compound f) is a vinylsiloxane, a vinyl ether or a compound having allyl groups.

9. The kit of parts according to Claim 7 or 8, **characterized in that** the catalyst g) is a platinum or palladium catalyst or a catalyst of an alloy of the two metals.

10. The kit of parts according to one of Claims 1-9, **characterized in that** the sub-kit of parts 1 contains
h) a copolymer without hydrosilyl groups.

11. The kit of parts according to one of Claims 1-10, **characterized in that** the sub-kit of parts 1 contains
i) a solvent.

12. The kit of parts according to one of Claims 1-11, **characterized in that** solvent I) is a readily volatile solvent.

13. The kit of parts according to one of Claims 1-12, **characterized in that** sub-kits of parts 1 and/or 2 contains biostatic, biocidal or other pharmaceutical compounds or agents.

14. The kit of parts according to one of Claims 1-13, **characterized in that** sub-kits of parts 2 contains fillers, thixotroping agents, thickeners, pigments, coloring agents, plasticizers, stabilizers, emulsifiers and/or hydrophobing agents.

15. A use of a kit of parts according to one of the preceding claims as an adhesive elastic sealing material.

16. The use of a kit of parts according to one of Claims 1-14 as an adhesive root canal cement.

17. The use of a kit of parts according to one of Claims 1-14 as an adhesive elastic dental filling material.

18. The use of a kit of parts according to one of Claims 1-14 as an adhesive elastic temporary dental filling material.

19. The use of a kit of parts according to one of Claims 1-14 as an elastic bone filling material.

20. The use of a kit of parts according to one of Claims 1-14 as an elastic bond.

21. The use of a kit of parts according to one of Claims 1-14 as an adhesive elastic cement.

22. The use of a kit of parts according to one of Claims 1-14 as an adhesive elastic temporary cement.

23. The use of a kit of parts according to one of Claims 1-14 as an elastic bracket adhesive.

24. The use of a kit of parts according to one of Claims 1-14 as an elastic splint cement.

25. The use of a kit of parts according to one of Claims 1-14 as an elastic bone cement.

## Revendications

1. Kit, qui contient
a) un copolymère avec des radicaux hydrogénosiloxane,
b) un catalyseur,
c) un composé vinyle, et
d) un composé hydrosilyle,
**caractérisé en ce que** le copolymère avec des radicaux hydrogénosiloxane a) est un copolymère de siloxane contenant des radicaux SiH avec des composés insaturés, ou
**en ce que** le copolymère avec des radicaux hydrogénosiloxane a) est un copolymère de composés insaturés, qui sont greffés avec des siloxanes contenant des radicaux SiH.

2. Kit selon la revendication 1, qui présente deux sous-kits, où
la sous-kit 1 contient
a) le copolymère avec des radicaux hydrogénosiloxane, et
la sous-kit 2 contient
b) le catalyseur,
c) le composé vinyle, et
d) le composé hydrosilyle.

3. Kit selon l'une quelconque des revendications 1 ou 2, **caractérisé en ce que** le catalyseur b) est un catalyseur au platine.

4. Kit selon l'une quelconque des revendications 1 à 3, **caractérisé en ce que** la sous-kit 1 contient
e) un composé hydrosilyle différent de a).

5. Kit selon la revendication 4, **caractérisé en ce que** le composé hydrosilyle e) est un polysiloxane avec au moins deux, de préférence trois radicaux Si-H.

6. Kit selon l'une quelconque des revendications 1 à 5, **caractérisé en ce que** le composé vinyle c) est un composé vinylsiloxane et le composé hydrosilyle d) est un composé hydrogénosiloxane.

7. Kit selon l'une quelconque des revendications 1 à 6, **caractérisé en ce que** le sous-kit 1 contient
f) un composé vinyle, et
g) un catalyseur.

8. Kit selon l'une quelconque des revendications 1 à 7, **caractérisé en ce que** le composé vinyle f) est un vinylsiloxane, un vinyléther ou un composé avec des radicaux allyle.

9. Kit selon la revendication 7 ou 8, **caractérisé en ce que** le catalyseur g) est un catalyseur au platine ou au palladium ou un catalyseur d'un alliage des deux métaux.

10. Kit selon l'une quelconque des revendications 1 à 9, **caractérisé en ce que** le sous-kit 1 contient
h) un (co)polymère sans radical hydrosilyle.

11. Kit selon l'une quelconque des revendications 1 à 10, **caractérisé en ce que** le sous-kit 1 contient
i) un solvant.

12. Kit selon l'une quelconque des revendications 1 à 11, **caractérisé en ce que** le solvant i) est un solvant facilement volatil.

13. Kit selon l'une quelconque des revendications 1 à 12, **caractérisé en ce que** les sous-kits 1 et/ou 2 contiennent des composés ou agents biostatiques, biocides ou pharmaceutiques autres.

14. Kit selon l'une quelconque des revendications 1 à 13, **caractérisé en ce que** le sous-kit 2 contient des charges, des agents thixotropes, des agents de renforcement, des pigments, des colorants, des plastifiants, des stabilisants, des émulsionnants et/ou des agent d'hydrophobisation.

15. Utilisation d'un kit selon l'une quelconque des revendications précédentes, comme agent de fermeture élastique adhérent.

16. Utilisation d'un kit selon l'une quelconque des revendications 1 à 14, comme ciment adhérent de canal racinaire.

17. Utilisation d'un kit selon l'une quelconque des revendications 1 à 14, comme matériau adhérent élastique d'obturation des dents.

18. Utilisation d'un kit selon l'une quelconque des revendications 1 à 14, comme matériau adhérent élastique provisoire d'obturation des dents.

19. Utilisation d'un kit selon l'une quelconque des revendications 1 à 14, comme matériau élastique d'obturation des os.

20. Utilisation d'un kit selon l'une quelconque des revendications 1 à 14, comme fixation élastique.

21. Utilisation d'un kit selon l'une quelconque des revendications 1 à 14, comme ciment adhérent élastique.

22. Utilisation d'un kit selon l'une quelconque des revendications 1 à 14, comme ciment adhérent élastique provisoire.

23. Utilisation d'un kit selon l'une quelconque des revendications 1 à 14, comme adhésif élastique de brackets.

24. Utilisation d'un kit selon l'une quelconque des revendications 1 à 14, comme fixation de goupille élastique.

25. Utilisation d'un kit selon l'une quelconque des revendications 1 à 14, comme ciment-colle élastique des os.
